(19) Europäisches Patentamt
European Patent Office
Office européen des brevets

(11) Veröffentlichungsnummer: **0 023 992**
**B1**

(12) # EUROPÄISCHE PATENTSCHRIFT

(45) Veröffentlichungstag der Patentschrift: **23.06.82**

(51) Int. Cl.³: **C 07 C 103/78**, A 61 K 49/04

(21) Anmeldenummer: **80104242.5**

(22) Anmeldetag: **18.07.80**

(54) **Neue Derivate der 2,4,6-Trijod-isophthalsäure, Verfahren zu deren Herstellung und diese enthaltende Röntgenkontrastmittel.**

(30) Priorität: **09.08.79 IT 2502779**

(43) Veröffentlichungstag der Anmeldung: **18.02.81 Patentblatt 81/7**

(45) Bekanntmachung des Hinweises auf die Patenterteilung: **23.06.82 Patentblatt 82/25**

(84) Benannte Vertragsstaaten: **AT BE CH DE FR GB LI NL SE**

(56) Entgegenhaltungen:
**DE-A-1 816 844**
**FR-A-2 293 919**
**FR-A-2 355 808**
**FR-A-2 385 698**

**Die Akte enthält technische Angaben, die nach dem Eingang der Anmeldung eingereicht wurden und die nicht in dieser Patentschrift enthalten sind.**

(73) Patentinhaber: **BRACCO INDUSTRIA CHIMICA Società per Azioni, Via E. Folli, 50, I-20134 Milano (IT)**

(72) Erfinder: **Felder, Ernst, Prof. Dr., Via Ceresio 49, CH-6826 Riva S. Vitale (CH)**
Erfinder: **Pitrè, Davide, Prof. Dr., Via Brocchi 24, Milano (IT)**

(74) Vertreter: **Zutter, Hans Johann Niklaus, EPROVA Aktiengesellschaft im Laternenacker 5, CH-8200 Schaffhausen (CH)**

EP 0 023 992 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Derivate der 2,4,6-Trijod-isophthalsäure, Verfahren zu deren Herstellung und diese enthaltende Röntgenkontrastmittel

Die vorliegende Erfindung betrifft die neuen, in Wasser leicht löslichen Bis[N-{3,5-bis-(hydroxyalkyl-aminocarbonyl)-2,4,6-trijod-phenyl}-N-(α-hydroxyacryl)-amino]-alkane der allgemeinen Formel (I)

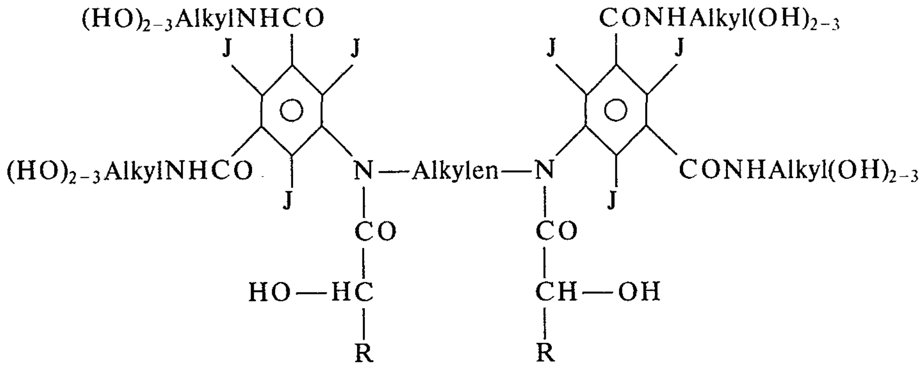

(I)

worin

$(HO)_{2-3}$Alkyl- 1,3-Dihydroxyisopropyl-, 2,3-Dihydroxypropyl- oder 1,3-Dihydroxy-2-hydroxymethylisopropyl-,

R Wasserstoff oder Methyl- und

Alkylen einen zweiwertigen Alkylen-Rest mit 2 bis 10 C-Atomen $[= -(CH_2)_{2-10}-]$, der durch Hydroxysubstituiert sein kann, oder einen Mono-, Di- oder Poly-oxaalkylen-Rest mit 4 bis 12 C-Atomen der allgemeinen Formel $-(C_nH_{2n}-O)_{1-4}-C_nH_{2n}-$, in der N = 2 oder 3 ist, der durch Hydroxy substituiert sein kann, wobei die Hydroxygruppen jeweils mit einer niedrigen Fettsäure, vorzugsweise Essigsäure, acyliert oder im Falle von zwei oder mehr Hydroxygruppen durch Acetal- oder Ketalbildung, vorzugsweise mit Aceton, maskiert sein können,

bedeutet, das Verfahren zu ihrer Herstellung sowie nicht ionische Röntgenkontrastmittel, die insbesondere zur Vasographie, Urographie, Bronchographie und zur Darstellung von Körperhöhlen und der Liquorräume geeignet sind und die vorstehend genannten Verbindungen als schattengebende Komponenten enthalten.

Beispiele für unsubstituierte Alkylen-Reste, welche die beiden Trijod-isophthalsäureamid-Moleküle verbinden, sind Äthylen, Propylen, Butylen, Pentylen, Hexylen, Octylen, Nonylen, Decylen. Im allgemeinen werden Alkyen-Reste mit 3 und mehr Kohlenstoffatomen vorgezogen, da sich solche leichter einfügen lassen.

Um den hydrophilen Charakter der Verbindungen zu verstärken, können in die Alkylen-Brücke, welche die beiden Molekülhälften zusammenhält. Sauerstoffatome eingebaut werden, und zwar indem man die Kohlenstoffkette jeweils nach 2 bis 3 Kohlenstoffatomen durch ein Sauerstoffatom unterbricht.

Beispiele für solche Sauerstoff-haltige Alkylenbrücken sind:

$$-CH_2CH_2-O-CH_2CH_2-$$

$$-CH_2CH_2-O-CH_2-CH_2-O-CH_2-CH_2-$$

$$-CH_2CH_2-O-CH_2-CH_2-O-CH_2-CH_2-O-CH_2-CH_2-$$

$$-CH_2-CH_2-(O-CH_2-CH_2)_3-O-CH_2-CH_2-$$

$$-CH_2CH_2-O-CH_2-CH_2-CH_2-O-CH_2-CH_2-$$

$$-CH_2CH_2CH_2-O-CH_2CH_2-O-CH_2CH_2CH_2-$$

$$-CH_2CH_2CH_2-O-CH_2CH_2CH_2-$$

Dem gleichen Zweck dient auch die Substitution einzelner H-Atome der Alkylen-Brücke durch Hydroxyl, wodurch beispielsweise folgende Alkylen-Reste entstehen

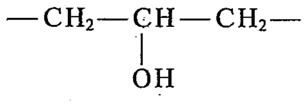 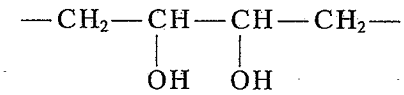

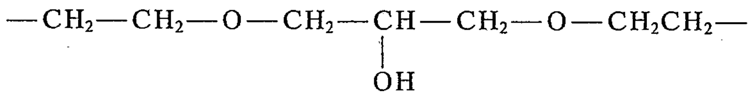

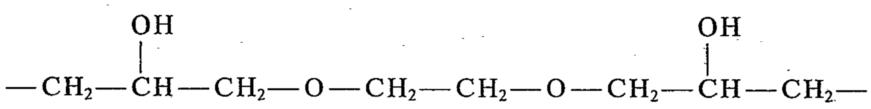

5-Acylamino-2,4,6-trijod-isophthalsäure-diamide und deren Anwendung in Röntgenkontrastmitteln sind aus der CH-PS 544 551 bekannt. Sie enthalten nur einfache, unsubstituierte aliphatische Acyl-Gruppen, in der Regel Acetyl-Gruppen. Einige Kohlenhydrat-Reste aufweisende Vertreter dieser Gruppe sind ausreichend wasserlöslich, beispielsweise das unter dem Freinamen METRIZAMIDE bekannt gewordene 3-Acetylamino-5-N-methyl-acetylamino-2,4,6-trijod-benzoylglucosamin. Vergleiche dazu: Verbindung Nr. 11 der US-PS 3 701 771, GB-PS 1 321 591, CH-PS 544 551, AT-PS 318 134 bzw. der DE-OS 2 031 724; Publikationen von T. Almen, S. Salvesen, K. Golman; Acta Radiologia Suppl. 335 (1973), 1—13, 233—75, 312—38. Nachteilig ist seine schwierige Zugänglichkeit und namentlich seine geringe Stabilität, welche die Verwendbarkeit wesentlich einschränkt und die Handhabung erschwert.

Einen Fortschritt stellt das unter dem Freinamen IOPAMIDOL bekannt gewordene L-5-$\alpha$-Hydroxy-propionylamino-2,4,6-trijod-isophthalsäure-bis-(1,3-dihydroxyisopropylamid) dar. Vergleiche dazu DE-PS 2 547 789, GB-PS 1 472 050, US-PS 4 001 323, Felder et al., Il FARMACO, Ed. Sc. 32 835—844 (1977). Es zeichnet sich aus durch eine wesentlich einfachere Struktur, durch höhere Stabilität, leichte Isolierbarkeit und durch eine geringere Viskosität seiner konzentrierten wäßrigen Lösungen. Die Toxizität dieser Verbindung ist sehr niedrig.

Zu den im europäischen Recherchenbericht zitierten Dokumenten wird bemerkt:

1. Die Verbindungen der DE-A-1 816 844 (PHAEMACIA), welche etwa durch Umsetzung von 5-Acylamino-2,4,6-trijod-N-alkylisophthalsäure-monoamid mit Bis-epoxyden erhalten werden, enthalten im Gegensatz zu den Verbindungen der vorliegenden Erfindung freie Carboxylgruppen und unsubstituierte Acyl-Reste, gewöhnlich Acetyl-Reste. Sie sind starke Säuren und nur als Salze ausreichend wasserlöslich und als Röntgenkontrastmittel verwendbar.
2. Die FR-A-2 385 698 (BRACCO) betrifft $\alpha,\omega$-Bis[5-dihydroxyalkyl)aminocarbonyl-3-hydroxyacylamino-2,4,6-trijodbenzoylamino]-oxaalkane.
3. In der FR-A-2 355 808 (SCHERING) sind vorwiegend Oxalsäure-bis-[3,5-bis(hydroxyalkyl-carbamoyl)-2,4,6-trijod-anilide] offenbart worden. In den Prioritätsunterlagen DE-OS 2 628 517 vom 23. 6. 1976 offengelegt am 5. 1. 1978 — sind sogar ausschließlich Oxalsäure-bis-anilide offenbart.

Die Entwicklung in den letzten Jahren hat deutlich gezeigt, daß es äußerst schwierig ist und nur selten gelingt nicht ionische Verbindungen zu finden, welche die ganz spezifischen Eigenschaften aufweisen, die für die einzelnen Techniken der Röntgenkontrastuntersuchungen erforderlich sind. Diese Eigenschaften sind echte Wasserlöslichkeit ausreichend zur Herstellung stabiler, d. h. nicht übersättigter konzentrierter Lösungen, maximale allgemeine und neurotrope Verträglichkeit, minimale Osmolalität, eine dem spezifischen Verwendungszweck angepaßte oftmals erhöhte Viskosität, maximale Stabilität gegen hydrolytische Einflüsse und ausreichend einfache Struktur zur Ermöglichung einer ökonomischen Synthese sowie zur Erleichterung der Isolierung und Reinigung.

Zu dieser auserlesenen Gruppe von schattengebenden Komponenten gehören auch die vorliegenden neuen Verbindungen der Formel (I).

Sie zeichnen sich im allgemeinen aus durch hohe Wasserlöslichkeit, die bei einzelnen Vertretern absolute Spitzenwerte erreicht, durch optimale Verträglichkeit und vor allem durch ihre geringe Osmolalität sowie ihrer durchwegs sehr großen Stabilität insbesondere gegenüber über hydrolytischen Einflüssen, worin sie die an sich schon gute Hydrolyse-Stabilität der ihnen zugrundeliegenden am aromatischen N-Atom nicht substituierten Ausgangsstoffe noch deutlich übertreffen. Diese verstärkte Stabilität gegen hydrolytische Einflüsse ist wichtig, zur Verhinderung der Bildung von Spuren freier aromatischer Amine im Hinblick auf mögliche aber unzulässige cytotoxische Effekte derselben in Verbindung mit Röntgenstrahlen. Vergleiche dazu: A. Norman et al., Radiology 129, 199—203 (Okt. 1978).

Besonders überraschend und zugleich wertvoll ist, daß durch die Verknüpfung von zwei Trijodisophthalsäureamid-Molekühle durch eine an das jeweilige aromatische N-Atom gebundene Alkylen-Brücke, die Wasserlöslichkeit der zugrundeliegenden Verbindung — zum Beispiel das 5-Hydroxyacetylamino-2,4,6-trijod-isophthalsäure-bis-(2,3-dihydroxypropylamid) — zum Teil erheblich verbessert wird.

Die erfindungsgemäßen Verbindungen zeichnen sich durch stark reduzierte Osmolalität und gewöhnlich durch erhöhte Viskosität aus, die für viele Anwendungszwecke insbesondere die Myelographie sowie beispielsweise für gewisse Untersuchungen an den Herzklappen, zur Darstellung von Körperhöhlen, zur Bronchographie, Hysterosalpingographie, Arthrographie und Lymphographie sehr erwünscht ist.

Das Hauptanwendungsgebiet der vorliegenden neuen Röntgenkontrastmittel ist die Neuroradiologie im besonderen die Darstellung der Hohlräume, welche die Cerebrospinal-Flüssigkeit enthalten. Diese Räume bestehen aus verschiedenen Cavitäten, die mit dem Zentralnervensystem verbunden sind. Sie umfassen z. B. die Ventrikel des Gehirnes, die Cisternae, den Subarachnoidalraum um das Gehirn herum, sowie den Spinalkanal. Die radiologische Untersuchung dieser Cavitäten wird gewöhnlich in drei Hauptgruppen unterteilt: Die Ventriculographie, die Cisternographie und die Myelographie. Die Myelographie ist die radiologsiche Untersuchung des spinalen Subarachnoidalraumes.

Die Verträglichkeit der Kontrastmittel, welche in diese Raäume eingebracht werden, ist gegenüber der intravenösen Verabreichung sehr stark herabgesetzt. Die Empfindlichkeit steigt je mehr man sich dem Gehirn nähert. Die verschiedenen Bereiche stehen jedoch in Kommunikation miteinander, wodurch ein Übertritt aus einem Gebiet in das benachbarte eventeull empfindlichere Gebiet möglich ist. Ein Hauptziel der röntgenologischen Inspektion ist die Feststellung raumbeengender Vorgänge etwa im Spinalkanal, die Abklärung von Funktionsstörungen durch fehlende oder gestörte Kommunikationen. Eine wichtige, aber besonders schwierige und für den Patienten belastende Untersuchung betrifft das Hohlraumsystem des Gehirns selbst.

Die Anforderungen an die physikalisch-chemischen Eigenschaften der Kontrastmittel wie angepaßte Viskosität zur leichten Ermöglichung der Applikation aber auch zur Verhinderung des vorzeitigen Abfließens aus den darzustellenden Hohlräumen und die richtige Osmolalität zur weitgehenden Ausschaltung von osmotischen Vorgängen sind besonders wichtig. Die Kontrastmittel sollen aber auch nicht zu lange im Spinalkanal liegen bleiben wie etwa die bislang verwendeten wasserunlöslichen, langsam metabolisierenden jodierten Öle. Schließlich sind die Ansprüche an die Verträglichkeit der Röntgenkontrastmittel in der Neuroradiologie verständlicherweise besonders hoch. Die Röntgenkontrastmittelmengen sind im Vergleich zu der Liquormenge im Inspektionsraum groß.

Die erfindungsgemäßen Verbindungen erfüllen die geforderten Bedingungen in optimaler Weise. Sie sind so viskos, daß sie lange genug in dem Inspektionsraum verweilen, um eine sichere röntgenologische Inspektion zu ermöglichen. Aufgrund ihrer Wasserlöslichkeit und Mischbarkeit mit der Cerebrospinalflüssigkeit bleiben sie aber auch nicht über lange Zeiträume an der Injektionsstelle liegen. Dank ihrer chemischen Stabilität werden sie nicht metabolisiert sondern im wesentlichen unverändert durch die Nieren ausgeschieden. Als nicht ionogene Kontrastmittel mit angepaßtem osmotischen Druck ihrer Lösungen geringer elektrischer Leitfähigkeit ist ihr Einfluß auf das nervöse Reizleitungssystem reduziert. Ihre Anwendung ist daher weniger schmerzhaft.

Das Verfahren zur Herstellung der als schattengebende Komponenten in Röntgenkontrastmitteln verwendbaren Bis-[N-{3,5-bis-(hydroxyalkylaminocarbonyl)-2,4,6-trijodphenyl}-N-(α-hydroxyacyl)-amino]-alkane der Formel I ist dadurch gekennzeichnet, daß man ein 5-α-Hydroxyacylamino-2,4,6-trijod-isophthalsäure-bis(dihydroxypropylamid) der allgemeinen Formel (III)

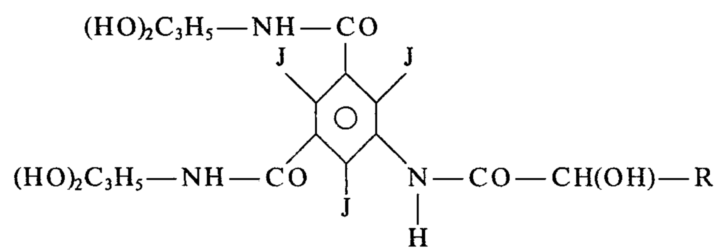

(III)

am an den Benzolkern gebundenen Stickstoff-Atom im alkalischen Milieu alkyliert durch Umsetzung mit Alkylierungsmitteln der allgemeinen Formel (IV)

X — Alkylen — X (IV)

wobei R und Alkylen sowie $(HO)_2C_3H_5-$ in den Formeln (III) bzw. (IV) dieselbe Bedeutung haben wie in der Formel I und X jeweils eines der Halogenatome Jod, Brom oder Chlor oder einen der Sulfat- bzw. Sulfonat-Reste $-OSO_2-OR_1$ bzw. $-OSO_2$-Alkyl oder $-OSO_2$-Aryl bedeutet.

Ein entsprechendes 5-α-Hydroxyacylamino-2,4,6-trijodisophthalsäure-bis-(dihydroxypropylamid) wird demnach in Gegenwart von Basen mit einem Alkylendisulfonat z. B. einem Bis-(methan-, benzol- oder Toluol-sulfonyloxy)-alkan umgesetzt.

Typische Beispiele für Alkylierungsmittel der Formel X-Alkylen-X sind:

1,2-Dijod-äthan, 1,2-Dibrom-äthan, 1,2-Dichlor-äthan, 1,3-Dichlor-propan, 1,3-Dijod-propan, 1,4-Dibrom-butan, 1,5-Dijod-pentan, 1,6-Dijod-hexan (Hexamethylendijodid), 1,7-Dibrom-heptan, 1,8-Dijod-octan, 1,9-Dibrom-nonan, 1,10-Dijod-decan, 1,5-Dichlor-3-oxa-pentan, 1,5-Dijod-3-oxa-pentan, 1,8-Dibrom-3,6-dioxa-octan, 1,11-Dijod-3,6,9-trioxa-undecan, 1,14-Dibrom-3,6,9,12-tetraoxa-tetradecan, 1,9-Dijod-3,7-dioxa-nonan, 1,10-Dibrom-4,7-dioxadecan, 1,7-Dibrom-4-oxaheptan, 1,4-Dibrom-2,3-bis-(acetoxy)-butan, 1,4-Dibrom-2,3-isopropylidendioxy-butan, 1,3-Dibrom-2-hydroxy-propan, 1,3-Dijod-2-acetoxy-propan, 1,9-Dibrom-3,7-dioxa-5-acetoxy-nonan, 1,10-Dijod-4,7-dioxa-2,9-bis-(acetoxy)-decan, 1,3-Bis-(methansulfonyloxy)-2-acetoxy-propan, 1,2-Bis-(4-toluolsulfonyloxy)-äthan, 1,3-Bis-(methansulfonyloxy)-propan, 1,4-Bis-(4-toluolsulfonyloxy)-butan, 1,5-Bis-(benzolsulfonyloxy)-butan, 1,16-Bis-(methansulfonyloxy)-4,7,10,13-tetraoxahexadecan, 1,3-Bis-(methoxysulfonyloxy)-propan, 1,4-Bis-(äthoxysulfonyloxy)-butan.

Wenn Alkylen-Brücken, die freie Hydroxygruppen enthalten, eingefügt werden, so ist es oft zweckmäßig, die Hydroxygruppe durch Acylierung mit einer niedrigen Fettsäure, wie z. B. Essigsäure oder im Falle von zwei oder mehr Hydroxygruppen durch Acetal- oder Ketalbildung z. B. mit Aceton zu maskieren und nach erfolgtem Umsatz mit dem entsprechenden 5-Acylamino-2,4,6-trijodisophthalsäure-diamid die Hydroxyl-Funktion durch Hydrolyse freizusetzen, wozu bei Acylderivaten Alkalien und bei Acetal- und Ketalderivaten Säuren verwendet werden.

Die bei der Alkylierung mit X-Alkylen-X frei werdende starke Säure (2 × HX) wird durch die anwesende Base abgefangen.

Als Basen kommen beispielsweise in Betracht: starke Alkalien wie z. B. Alkalialkoholate (NaOMe, NaOEt, KOMe, KOEt, LiOMe, LiOEt), Alkalihydroxyde (NaOH, KOH, LiOH), Alkalicarbonate ($Na_2CO_3$, $K_2CO_3$), quaternäre Ammoniumhydroxyde (Tetramethylammoniumhydroxyd). Die Umsetzung wird gewöhnlich in einem polaren Lösungsmittel wie z. B. in Wasser, niedrigen Alkoholen (MeOH, EtOH, Äthylenglykol, Propylenglykol, Glycerin), niedrigen Glykoläthern (Methoxyäthanol, Äthoxyäthanol, Butyloxyäthanol), Ketonen (Aceton, Methyläthylketon, Methylisopropylketon, Methylisobutylketon) oder in ausgesprochen aprotischen Lösungsmitteln wie z. B. Hexametapol (MPT), Dimethylformamid (DMF), Dimethylacetamid (DMAC), Dimethylsulfoxyd (DMSO), oder in Lösungsmittelgemischen durchgeführt. Durch Erwärmen wir die Umsetzung beschleunigt.

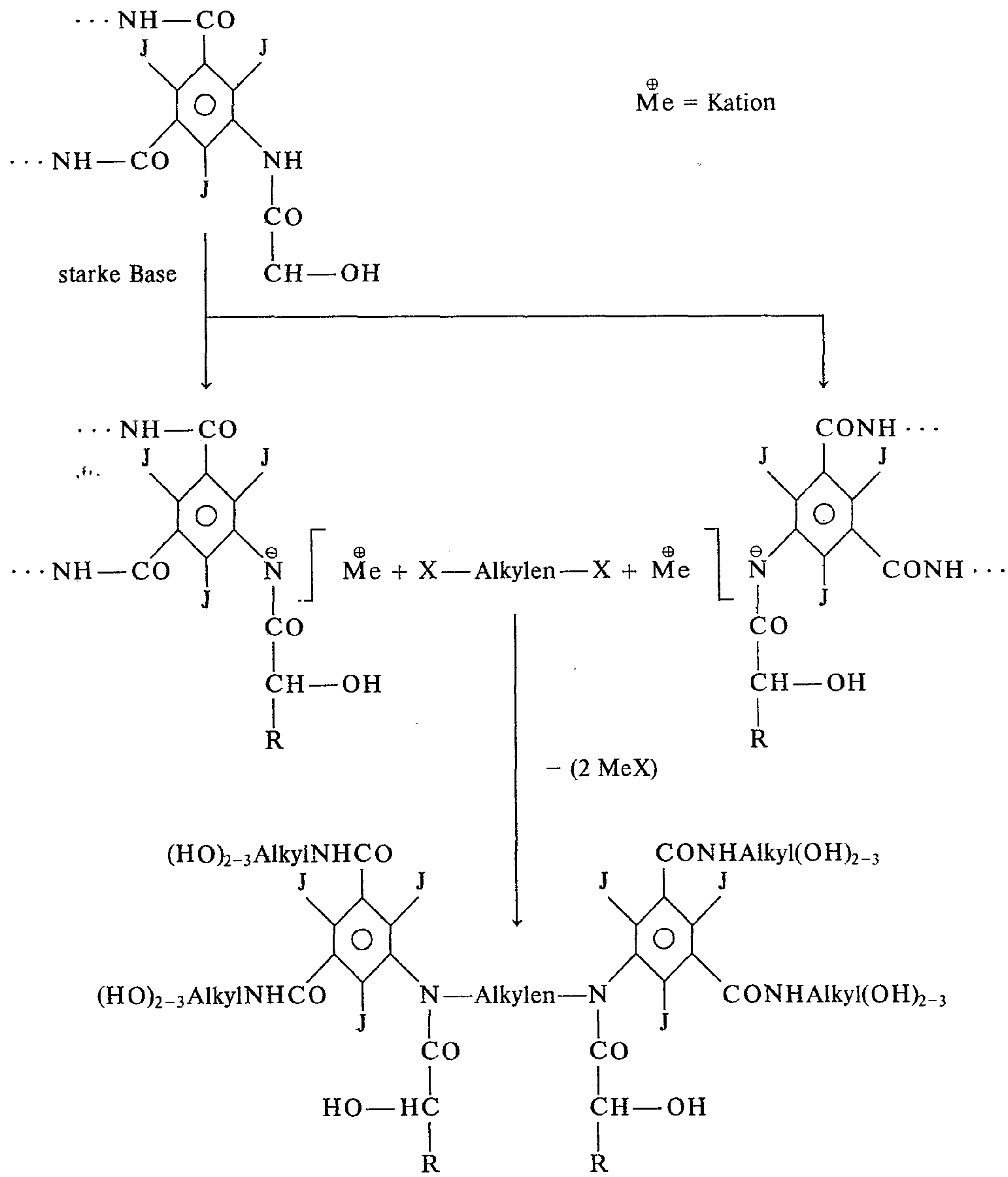


**Beispiele**

**Beispiel 1**

**1,6-Bis-[N-{3,5-bis-(1,3-dihydroxyisopropylaminocarbonyl)-2,4,6-trijod-phenyl}-N-(L-α-hydroxypropyionyl)-amino]-hexan**

Formel II: $(HOH)_2C_3H_5 = (HOCH_2)_2CH$, $R = -CH_3$, Alkylen $= -(CH_2)_6-$

58,3 g L-5-α-Hydroxypropionyl-amino-2,4,6-trijod-isophthalsäure-bis-(1,3-dihydroxyisopropylamid) [IOPAMIDOL] (0,075 Mol) werden in 200 ml Wasser gelöst und mit genau der stöchiometrischen Menge (0,075 Mol) 2N NaOH versetzt. Die Lösung hat ein pH von 11,9. Sie wird im Vakuum zur Trockne eingedampft. Der Rückstand bestehend aus der 5 N-Natrium-Verbindung (Na-Salz) von l-5-α-Hydroxypropionylamino-2,4,6-trijod-isophthalsäure-bis-(1,3-dihydroxyisopropylamid) wird im Vakuum bei 100° C getrocknet.

Äquivalentgewicht für $C_{17}H_{21}J_3N_3NaO_8$:
ber. 799,27
gef. 799,08.

Das so erhaltene Na-Salz (60 g = 0,075 Mol) wird in 180 ml Dimethylacetamid (DMAC) gelöst und bei 30°C tropfenweise mit 12,8 g Hexamethylenjodid (0,037 Mol) versetzt und bis zur vollständigen Umsetzung bei 45 – 50°C gerührt.

Die Reaktionslösung wird im Vakuum eingedampft. Der ölige Rückstand wird mit 300 ml Methylenchlorid versetzt. Der entstandene Niederschlag wird abfiltriert, wiederholt in absolutem Äthanol gewaschen, in 250 ml Wasser gelöst und mit Hilfe von Ionenaustauscherharzen entsalzt und anschließend mittels Perkolation durch eine mit Adsorberharz (Agglomeraten aus Styrolpolymerisaten) beschickten Säule weiter gereinigt.

Das Eluat wird eingedampft. Der ölige Rückstand kristallisiert beim Behandeln mit siedendem Äthanol.

Ausbeute: 50 g 1,6-Bis-[N-{3,5-bis-(1,3-dihydroxyisopropylaminocarbonyl)-2,4,6-trijod-phenyl}-N-(L-α-hydroxypropionyl)-amino]-hexan, das sind 81% der Theorie.
Schmelzpunkt: 285 – 290°C (Erweichung bei 195°C).
DC: Rf = 0,20; 0,30 und 0,41. Laufmittel $CH_2Cl_2/MeOH = 2:1$.
$C_{40}H_{54}J_6N_6O_{16}$: J ber. 46,53%; gef. 46,35%.
$[\alpha]_D^{20} = +13{,}93°$ $[\alpha]_{436}^{20} = +34{,}04°$ (c = 1% in Wasser).
Wasserlöslichkeit: ≥ 100% (g/v) bei 25°C.

## Beispiel 2

1,7-Bis-[N-{3,5-bis-(1,3-dihydroxyisopropylaminocarbonyl)-2,4,6-trijod-phenyl}-N-(L-α-hydroxypropionyl)-amino]-heptan

Formel II: $(HO)_2C_3H_5 = (HOCH_2)_2CH$, R = $-CH_3$, Alkylen = $-(CH_2)_7-$

80 g Na-Salz von IOPAMIDOL (0,1 Mol) in 180 ml DMAC werden mit 17,6 g 1,7-Dijodheptan (0,05 Mol) ähnlich wie im Beispiel 1 beschrieben umgesetzt und aufgearbeitet.

Die weitere Reinigung des mit Ionenaustauscherharzen entsalzten Rohproduktes erfolgt durch Gegenstromverteilung (Liquid-Liquid-Extraktion) zwischen Wasser (10 × 400 ml) und n-Butanol (10 × 400 ml).

Schmelzpunkt: ca. 220°C.
DC: Rf = 0,14 und 0,19. Laufmittel $CH_2Cl_2/MeOH = 2:1$.
$C_{41}H_{56}J_6N_6O_{16}$: J ber. 46,14%; gef. 46,34%.
$[\alpha]_{436}^{20} = 38°$ (c = 5% in Wasser).
Wasserlöslichkeit: 20% (g/v) bei 20°C, 100% (g/v) bei Siedetemperatur.

## Beispiel 3

1,9-Bis-[N-{3,5-bis-(1,3-dihydroxyisopropylaminocarbonyl)-2,4,6-trijod-phenyl}-N-(L-α-hydroxypropionyl)-amino]-nonan

Formel II: $(HO)_2C_3H_5- = (HOCH_2)_2CH-$, R = $-CH_3$, Alkylen = $-(CH_2)_9-$

90 g Na-Salz von IOPAMIDOL (0,112 Mol) in 200 ml DMAC werden mit 21,4 g 1,9-Dijodnonan (0,0564 Mol) bei 20 – 30°C ähnlich wie im Beispiel 2 umgesetzt und aufgearbeitet.

Die erhaltene Titelverbindung schmilzt bei ca. 230°C (Zers.).

DC: Rf = 0,19 und 0,26. Laufmittel $CH_2Cl_2/MeOH = 2:1$.
$C_{43}H_{60}J_6N_6O_{16}$: J ber. 45,36%; gef. 45,34%.
$[\alpha]_{436}^{20} = 44°$ (c = 2% in Wasser).
Wasserlöslichkeit: 40% (g/v) bei 25°C.

## Beispiel 4

1,7-Bis-[N-{3,5-bis-(1,3-dihydroxyisopropylaminocarbonyl)-2,4,6-trijod-phenyl}-N-hydroxyacetyl-amino]-heptan

Formel II: $(HO)_2C_3H_5- = (HOCH_2)_2CH-$, R = $-H$, Alkylen = $-(CH_2)_7-$

51 g Na-Salz von 5-Hydroxyacetylamino-2,4,6-trijod-isophthalsäure-bis-(1,3-dihydroxyisopropylamid) (0,065 Mol) — hergestellt aus der freien Verbindung durch Umsetzung mit der äquivalenten

Menge Natriummethylat in Methanol — werden in Methanol oder Methoxyäthanol (150—200 ml) mit 11,5 g 1,7-Dijodheptan (0,0325 Mol) umgesetzt.

Ausbeute: 35 g 1,7-Bis-[N-{3,5-bis-(1,3-dihydroxyisopropylaminocarbonyl)-2,4,6-trijod-phenyl}-N-hydroxyacetyl-amino]-heptan, das sind 66% der Theorie.
Schmelzpunkt. 285—290° C unter Zersetzung.
DC: Rf = 0,05. Laufmittel $CH_2Cl_2/MeOH = 2:1$
= 0,04. Laufmittel $CHCl_3/MeOH/NH_4OH$ (25%) = 6 : 3 : 1.
$C_{41}H_{56}J_6N_6O_{16}$: J ber. 46,92%; gef. 46,70%.
Wasserlöslichkeit: 20% (g/v) bei 25° C, 100% (g/v) bei Siedetemperatur.

## Beispiel 5

### 1,9-Bis-[N-{3,5-bis-(1,3-dihydroxyisopropylaminocarbonyl)-2,4,6-trijod-phenyl}-N-hydroxyacetyl-amino]-nonan

Formel II: $(HO)_2C_3H_5- = (HOCH_2)_2CH-$, R = —H, Alkylen = $-(CH_2)_9-$

44 g Na-Salz von 5-Hydroxyacetylamino-2,4,6-trijod-isophthalsäure-bis-(1,3-dihydroxyisopropyl-amid) — hergestellt aus der freien Verbindung durch Umsetzung mit der äquivalenten Menge Natriummethylat in Methanol — werden in Methanol, Methoxyäthanol oder DMAC (200 ml) mit 10,6 g 1,9-Dijodnonan (0,028 Mol) umgesetzt. Eine potentiometrische Jodid-Titration zeigt, daß die Umsetzung schon nach ziemlich kurzer Zeit quantitativ vollzogen ist.

Ausbeute: 38 g 1,9-Bis-[N-{3,5-bis-(1,3-dihydroxyisopropylaminocarbonyl)-2,4,6-trijod-phenyl}-N-hydroxyacetyl-amino]-nonan, das sind 82% der Theorie.
Schmelzpunkt: 180—182° C.
DC: Rf = 0,1 · Laufmittel $CH_2CL_2/MeOH = 2:1$
= 0,06. Laufmittel $CHCl_3/MeOH/NH_4OH$ (25%) = 6 : 3 : 1.
$C_{41}H_{56}J_6N_6O_{16}$: J ber. 46,14%; gef. 46,58%.
Wasserlöslichkeit: ≥ 100% (g/v) bei 25° C.

## Beispiel 6

### 1,9-Bis-[N-{3,5-bis-(2,3-dihydroxypropylaminocarbonyl)-2,4,6-trijod-phenyl}-N-(L-α-hydroxypropionyl)-amino]-nonan

Formel II: $(HO)_2C_3H_5- = HOCH_2CH(OH)-CH_2-$, R = $-CH_3$, Alkylen = $-(CH_2)_9-$

51 g K-Salz von L-5-α-Hydroxypropionylamino-2,4,6-trijodisophthalsäure-bis-(2,3-dihydroxypropyl-amid) (0,0625 Mol) — hergestellt durch Umsetzung von 48,6 g der freien Verbindung mit 31,25 ml 2N KOH und Eindampfen der gebildeten Lösung und Trocknen des Rückstandes — werden mit 14,4 g 1,9-Dijodnonan in 150 ml Dimethylsulfoxyd umgesetzt.

Ausbeute: 47 g 1,9-Bis-[N-{3,5-bis-(2,3-dihydroxypropylaminocarbonyl)-2,4,6-trijod-phenyl}-N-(L-α-hydroxypropionyl)-amino]-nonan, das sind 90% der Theorie.
Schmelzpunkt: 187° C.
DC: Rf = 0,08 und 0,10. Laufmittel $CH_2Cl_2/MeOH = 2:1$.
$C_{43}H_{60}J_6N_6O_{16}$: J ber. 45,36%; gef. 44,99%.
$[\alpha]^{20}_{436}$ = +38,3° (c = 1% in Wasser).
Wasserlöslichkeit: ≥ 100% (g/v) bei 25° C.

## Beispiel 7

### 1,8-Bis-[N-{3,5-bis-(1,3-dihydroxyisopropylaminocarbonyl)-2,4,6-trijod-phenyl}-N-(L-α-hydroxypropionyl)-amino]-3,6-dioxa-octan

Formel II: $(HO)_2C_3H_5- = (HOCH_2)_2CH-$, R = $-CH_3$, Alkylen = $-CH_2CH_2-O-CH_2CH_2-O-CH_2CH_2-$

80 g Na-Salz von IOPAMIDOL (0,1 Mol) werden in 200 ml DMAC mit 1,8-Dijod-3,6-dioxa-octan (0,051 Mol) umgesetzt und wie in den vorstehenden Beispielen beschrieben aufgearbeitet und durch

Gegenstromverteilung (Wasser/Butanol) weiter gereinigt und schließlich durch Lösen in siedendem Äthanol zur Kristallisation gebracht.

Ausbeute: 54,3 g 1,8-Bis-[N-{3,5-bis-(1,3-dihydroxyisopropylaminocarbonyl)-2,4,6-trijod-phenyl}-N-(L-α-hydroxypropionyl)-amino]-3,6-dioxa-octan, das sind 65% der Theorie.
Schmelzpunkt: 127—128° C.
DC: Rf=0,16; 0,23 und 0,30. Laufmittel: $CH_2Cl_2$/MeOH=2 : 1.
$C_{40}H_{54}J_6N_6O_{18}$: J ber. 45,63%; gef. 45,44%.
$[\alpha]^{20}_{436}$ = +50,0° (c=1% in Wasser).
Wasserlöslichkeit: 100% (g/v) bei 25° C.

## Beispiel 8

1,11-Bis-[N-{3,5-bis-(1,3-dihydroxyisopropylaminocarbonyl)-2,4,6-trijod-phenyl}-N-(L-α-hydroxypropionyl)-amino]-3,6,9-trioxa-undecan

Formel II: $(HO)_2C_3H_5$— =$(HOCH_2)_2CH$—, R= —$CH_3$,
Alkylen= —$CH_2CH_2$—O—$CH_2CH_2$—O—$CH_2CH_2$—O—$CH_2CH_2$—

80 g Na-Salz von IOPAMIDOL (0,1 Mol) werden mit 22 g 1,11-Dijod-3,6,9-trioxa-undecan (0,053 Mol) umgesetzt.

Ausbeute: 59,7 g 1,11-Bis-[N-{3,5-bis-(1,3-dihydroxyisopropylaminocarbonyl)-2,4,6-trijod-phenyl}-N-(L-α-hydroxypropionyl)-amino]-3,6,9-trioxa-undecan, das sind 70% der Theorie.
Schmelzpunkt: 185° C.
DC: Rf=0,21; 0,29 und 0,34. Laufmittel: $CH_2CL_2$/MeOH=2 : 1.
$C_{42}H_{58}J_6N_6O_{19}$: J ber. 44,46%; gef. 44,58%.
$[\alpha]^{20}_{436}$ = +43,78° (c=1% in Wasser).
Wasserlöslichkeit: ≥100% (g/v) bei 25° C.

## Beispiel 9

1,5-Bis-[N-{3,5-bis-(1,3-dihydroxyisopropylaminocarbonyl)-2,4,6-trijod-phenyl}-N-(L-α-hydroxypropionyl)-amino]-3-oxa-pentan

Formel II: $(HO)_2C_3H_5$=$(HOCH_2)_2CH$—, R= —$CH_3$, Alkylen= —$CH_2CH_2$—O—$CH_2CH_2$—

119,9 g Na-Salz von IOPAMIDOL (0,15 Mol) in 210 ml Dimethylsulfoxyd werden bei 20—25° C mit 28,6 g 1,5-Dijod-3-oxa-pentan versetzt und bei Raumtemperatur etwa 4—5 Tage gerührt. Die gebildete neue Verbindung wird durch Versetzen der Reaktionslösung mit Methylenchlorid ausgefällt und mittels Perkolation durch mit Ionenaustauscherharzen beschickte Säulen entsalzt.

Man erhält 79,8 g 1,5-Bis-[N-{3,5-bis-(1,3-dihydroxyisopropylaminocarbonyl)-2,4,6-trijod-phenyl}-N-(L-α-hydroxypropionyl)-amino]-3-oxa-pentan, das sind 65,5% der Theorie.
Schmelzpunkt: 265° C Zersetzung.
DC: Rf=0,34. Laufmittel $CH_2Cl_2$/MeOH=2 : 1.
$[\alpha]^{20}_{C}$ = +19,67°, $[\alpha]^{20}_{436}$ =43,84°, $[\alpha]^{20}_{365}$ = +81,12°; c=1% in Wasser
Löslichkeiten: spielend leicht löslich in Wasser, Methanol und Äthanol.

## Beispiel 10

1,8-Bis-[N-{3,5-bis-(1,3-dihydroxyisopropylaminocarbonyl)-2,4,6-trijod-phenyl}-N-hydroxyacetylamino]-3,6-dioxa-octan

Formel II: $(HO)_2C_3H_5$=$(HOCH_2)_2CH$—, R= —H, Alkylen= —$CH_2CH_2$—O—$CH_2CH_2$—O—$CH_2CH_2$—

117,6 g Na-Salz von 5-Hydroxyacetylamino-2,4,6-trijodisophthalsäure-bis-(1,3-dihydroxyisopropyl-amid) (0,15 Mol) in 200 ml Dimethylsulfoxyd werden mit 28,6 g 1,8-Dijod-3,6-dioxaoctan versetzt und bei Raumtemperatur etwa 3 Tage gerührt bis sich die für vollständigen Umsatz berechnete Menge von Natriumjodid gebildet hat. Die neue Verbindung wird nach der im Beispiel 9 beschriebenen Methode isoliert und gereinigt.

Ausbeute: 92,3 g 1,8-Bis-[N-{3,5-bis-(1,3-dihydroxyisopropylaminocarbonyl)-2,4,6-trijod-phenyl}-N-hydroxyacetylamino]-3,6-dioxa-octan, das sind 75% der Theorie.
Schmelzpunkt: 240° C Zersetzung.
DC: 0,24 und 0,44. Laufmittel $CHCl_3/MeOH = 1 : 1$.
Löslichkeiten: Spielend leicht löslich in Wasser, löslich in siedendem Methanol und Äthanol, bei Raumtemperatur wenig löslich in Methanol und Äthanol.

Das als Zwischenprodukt verwendete 5-Hydroxyacetylamino-2,4,6-trijod-isophthalsäure-bis-(1,3-dihydroxyisopropylamid) wird nach in der DE-PS 2 547 789 beschriebenen Methode wie folgt erhalten:

A) 5-Amino-2,4,6-trijod-isophthalsäure-dichlorid (59,6 g) wird in DMAC mit 34 g Acetoxyacetylchlorid (0,25 Mol) umgesetzt, wobei 67,5 g 5-Acetoxyacetylamino-2,4,6-trijod-isophthalsäure-dichlorid vom Schmelzpunkt 234—235° C erhalten werden.

B) 150 g 4-Acetoxyacetylamino-2,4,6-trijod-isophthalsäure-dichlorid werden in 810 ml DMAC mit 80 g Tributylamin und danach tropfenweise mit 49,2 g Serinol [=1,3-Dihydroxyisopropylamin] in 540 ml DMAC versetzt. Man erhält 172 g 5-Acetoxyacetylamino-2,4,6-trijodisophthalsäure-bis-(1,3-dihydroxyisopropylamid), welches etwa 190—192° C unter Zersetzung schmilzt. Diese Verbindung wird in Wasser suspendiert und bei 45° C vorsichtig mit 1 N NaOH bei pH 11 behandelt, bis die Acetoxy-Gruppe vollständig hydrolysiert ist.

Die erhaltene Lösung wird durch Perkolation durch eine mit Kationenaustauscherharz (Amberlite® IR 120) und eine mit Anionenaustauscherharz (Amberlite® IR 45) beschickte Säule entsalzt. Das Eluat wird zur Trockene eingedampft und in 90%igem Äthanol aufgenommen, wobei das gewünschte Zwischenprodukt 5-Hydroxyacetylamino-2,4,6-trijod-isophthalsäure-bis-(1,3-dihydroxyisopropylamid) (73 g) kristallin anfällt.
Schmelzpunkt 300° C unter Zersetzung.

## Beispiel 11

### 1,5-Bis-[N-{3,5-bis-(1,3-dihydroxyisopropylaminocarbonyl)-2,4,6-trijod-phenyl}-N-hydroxyacetyl-amino]-3-oxa-pentan

Formel II: $(HO)_2C_3H_5- = (HOCH)_2CH-$, $R = -H$, Alkylen $= -CH_2CH_2-O-CH_2CH_2-$

117,6 g Na-Salz von 5-Hydroxyacetylamino-2,4,6-trijodisophthalsäure-bis-(1,3-dihydroxyisopropylamid) — hergestellt nach der im Beispiel 1 beschriebenen Methode — in 200 g Dimethylsulfoxyd (DMSO) werden mit 24,5 g 1,5-Dijod-3-oxa-pentan versetzt und 3 Tage bei Raumtemperatur gerührt.

Nach der Aufarbeitung erhält man 75,2 g der Titelverbindung, das sind 63% der Theorie.
Schmelzpunkt: 245° C.
DC: Rf = 0,27. Laufmittel $CHCl_3/MeOH = 1 : 1$.
$C_{36}H_{46}J_6N_6O_{17}$: J ber. 47,70%; gef. 48,00%.
Löslichkeiten: Diese Verbindung ist spielend leicht löslich in Wasser, dagegen nur beschränkt löslich in Methanol und Äthanol.

## Beispiel 12

### 1,4-Bis-[N-{3,5-bis-(1,3-dihydroxyisopropylaminocarbonyl)-2,4,6-trijod-phenyl}-N-(L-α-hydroxypropionyl)-amino]-2,3-dihydroxybutan

Formel II: $(HO)_2C_3H_5 = (HOCH_2)_2CH-$, $R = -CH_3$, Alkylen $= -CH_2-CHOH-CHOH-CH_2-$

78,5 g Na-Salz von IOPAMIDOL (~0,1 Mol) gelöst in 150 ml Dimethylsulfoxid werden bei 20—25° C mit 16,6 g 1,4-Dibrom-2,3-bis(acetoxy)-butan versetzt und während 90—100 Stunden bei Raumtemperatur gerührt. Die Umsetzung kann mit Hilfe argentometrischer Titration des gebildeten Natriumbromids verfolgt werden.

Nach vollendeter Umsetzung wird das gebildete Produkt durch Zusatz von 250 ml Methylenchlorid ausgefällt. Die Fällung wird in 500 ml Wasser gelöst und bei 50° C vorsichtig mit 2 N Natronlauge bei pH 10 behandelt, bis die Acetoxy-Funktionen in 2,3-Stellung der Alkylen-Brücke vollständig hydrolysiert sind.

Die erhaltene Lösung wird mit Hilfe von Ionenaustauscherharzen entsalzt. Das entsalzte Eluat wird eingedampft. Der Rückstand wird in Wasser gelöst, die Lösung durch ein Millipore®-Filter(Porengröße 0,45 mμ) gepreßt. Das Filtrat wird erneut zur Trockene eingedampft.

Man erhält 53 g der Titelverbindung, das sind 65% der Theorie.
Schmelzpunkt: 210—225° C unter Zersetzung.
$C_{38}H_{50}J_6N_6O_{18}$: J ber. 46,42%, gef. 46,25%.
Wasserlöslichkeit: ≥100% (g/v) bei 25° C.

Beispiel 13

1,5-Bis-[N-{3,5-bis-(2,3-dihydroxypropylaminocarbonyl)-2,4,6-trijod-phenyl}-N-hydroxyacetyl-amino]-3-oxapentan

Formel II: $(HO)_2C_3H_5 = HOCH_2-CHOH-CH_2-$, R = —H, Alkylen = $-CH_2CH_2-O-CH_2CH_2-$

117,6 g Na-Salz von 5-Hydroxyacetylamino-2,4,6-trijodisophthalsäure-bis-(2,3-dihydroxypropylamid) — hergestellt analog Beispiel 1 — werden in 200 g DMSO mit 24,5 g 1,5-Dijod-3-oxa-pentan analog Beispiel 11 umgesetzt.

Nach der Aufarbeitung erhält man 82,0 g der Titelverbindung, das sind 68% der Theorie.
Schmelzpunkt: >200° C unter Zersetzung.
DC: Rf = 0,15. Laufmittel $CHCl_3$/MeOH = 1 : 1.
$C_{36}H_{46}J_6N_6O_{17}$: J ber. 47,70%; gef. 47,48%.

Diese Verbindung ist spielend leicht löslich in kaltem Wasser, löslich in siedendem Methanol, dagegen nur wenig löslich in kaltem Methanol und in Äthanol.
Das als Zwischenprodukt verwendete 5-Hydroxyacetylamino-2,4,6-trijod-isophthalsäure-bis-(2,3-dihydroxypropylamid) wird nach der in der DE-PS 2 457 789 beschriebenen Methode wie folgt erhalten:
24,4 g 5-Acetoxyacetylamino-2,4,6-trijod-isophthalsäuredichlorid (0,035 Mol) gelöst in 60 ml DMAC werden unter Rühren zu einer Lösung von 15,9 g 2,3-Dihydroxypropylamin [= 1-Amino-2,3-dihydroxypropan] (0,175 Mol) in 100 ml DMAC getropft.
Man erhält öliges 5-Acetoxyacetylamino-2,4,6-trijodisophthalsäure-bis-(2,3-dihydroxypropylamid). Diese Verbindung wird in 250 ml Wasser aufgenommen und bei 40° C vorsichtig mit 1 N NaOH behandelt, bis die Acetoxy-Gruppe vollständig hydrolysiert ist.
Die erhaltene Lösung wird durch Perkolation durch eine mit Kationenaustauscherharz (Amberlite® IR 120) und eine mit Anionenaustauscherharz (Amberlite® IR 45) beschickte Säule entsalzt. Das Eluat wird eingedampft. Nach einiger Zeit tritt Kristallisation ein. Durch Umkristallisieren aus wenig Wasser wird das gewünschte Zwischenprodukt 5-Hydroxyacetylamino-2,4,6-trijod-isophthalsäure-bis-(2,3-dihydroxypropylamid) (19,4 g) in reiner Form erhalten.

Schmelzpunkt: 290° C.
DC: RF = 0,24; Laufmittel: Äthylacetat/Äthanol/Ammoniak (25%ig) = 15 : 7 : 6.
$C_{16}H_{20}J_3N_3O_8$: C ber. 25,18%; gef. 25,01%; J ber. 49,89%; gef. 49,75%.

Beispiel 14

1,8-Bis-[N-{3,5-bis-(2,3-dihydroxypropylaminocarbonyl)-2,4,6-trijod-phenyl}-N-hydroxyacetylamino]-3,6-dioxaoctan

Formel II: $(HO)_2C_3H_5- = HOCH_2-CH(OH)-CH_2-$ R = —H, Alkylen = $-CH_2CH_2OCH_2CH_2OCH_2CH_2-$

117,5 g Na-Salz von 5-Hydroxyacetylamino-2,4,6-trijodisophthalsäure-bis-(2,3-dihydroxypropylamid) (0,15 Mol) werden analog Beispiel 11 mit 28,6 g (0,075 Mol) 1,8-Dijod-3,5-dioxa-octan umgesetzt.

Man erhält 73,5 g der Titelverbindung, das sind 60% der Theorie.
Schmelzpunkt: >200° C unter Zersetzung.
DC: Rf = 0,14. Laufmittel Chloroform/MeOH = 1 : 1.
$C_{38}H_{50}J_6N_6O_{18}$ J ber. 46,42%; gef. 46,65%.

Diese Verbindung ist spielend leicht löslich in kaltem Wasser, leicht löslich in siedendem Methanol, dagegen nur beschränkt löslich in kaltem Methanol und in siedendem Äthanol.

Beispiel 15

1,3-Bis-[N-{3,5-bis-(2,3-dihydroxypropylaminocarbonyl)-2,4,6-trijodphenyl}-N-hydroxyacetyl-amino]-propan

Formel II: $(HO)_2C_3H_5— = HOCH_2—CHOH—CH_2—$, R = —H, Alkylen = $—CH_2—CH_2—CH_2—$

52,3 g Na-Salz von 5-Hydroxyacetylamino-2,4,6-trijodisophthalsäure-bis-(2,3-dihydroxypropylamid) (0,067 Mol) werden bei 45°C in 150 ml DMAC gelöst und bei Raumtemperatur mit 6,75 g 1,3-Dibrompropan (0,034 Mol) versetzt und bis zur vollständigen Umsetzung einige Stunden gerührt. Die Aufarbeitung erfolgt nach der im Beispiel 1 beschriebenen Methode.

Man erhält 37,2 g der Titelverbindung, das sind 71,2% der Theorie.
Schmelzbereich: 234—236° C unter Zersetzung.
DC: Rf = 0,27. Laufmittel 2-Butanon/AcOH/$H_2O$ = 15 : 3 : 5.
$C_{35}H_{44}J_6N_6O_{18}$: J ber. 48,61%; gef. 48,26%.

Diese Verbindung ist spielend leicht löslich in Wasser [100% (g/v) bei 25° C], beschränkt löslich in Methanol [3,3%], wenig löslich in Äthanol und Chloroform.

Beispiel 16

1,3-Bis-[N-{3,5-bis-(1,3-dihydroxyisopropylaminocarbonyl)-2,4,6-trijod-phenyl}-N-(L-α-hydroxypropionyl)-amino]-propan

Formel II: $(HO)_2C_3H_5— = (HOCH_2)_2CH—$, R = $—CH_3$, Alkylen = $—CH_2—CH_2—CH_2—$

59,9 g Na-Salz von IOPAMIDOL (0,075 Mol) werden in 105 g DMSO bei Raumtemperatur mit 11,5 g 1,3-Dijodpropan (0,0375 Mol) umgesetzt.

Man erhält 31 g der Titelverbindung, das sind 52% der Theorie.
Schmelzbereich: 280° C.
DC: Rf = 0,29. Laufmittel Methylenchlorid/Methanol = 2 : 1.
$C_{37}H_{48}J_6N_6O_{16}$: J ber. 47,76%; gef. 47,54%.

Die Verbindung ist spielend leicht löslich in Wasser [100% (g/v) bei 25° C], leicht löslich in Methanol [25% (g/v) bei 25°C und 100% (g/v) bei Siedetemperatur], dagegen nur sehr beschränkt löslich in Äthanol [1% bei 25° C] und praktisch unlöslich in Chloroform.

Beispiel 17

1,7-Bis-[N-{3,5-bis-(2,3-dihydroxypropylaminocarbonyl)-2,4,6-trijodphenyl}-N-hydroxyacetyl-amino]-4-oxaheptan

Formel II: $(HO)_2C_3H_5— = HOCH_2—CHOH—CH_2—$, R = —H, Alkylen = $—CH_2CH_2CH_2OCH_2CH_2CH_2—$

125,5 g Na-Salz von 5-Hydroxyacetylamino-2,4,6-trijodisophthalsäure-bis-(2,3-dihydroxypropylamid) (0,16 Mol) in 210 g DMSO werden unter Rühren bei Raumtemperatur mit 28,4 g 1,7-Dijod-4-oxaheptan umgesetzt.

Man erhält 104 g der Titelverbindung, das sind 80% der Theorie.
Schmelzbereich: 225° sintern, 250° C Zersetzung.
DC: Rf 0,15. Laufmittel Chloroform/Methanol = 1 : 1.
$C_{38}H_{50}J_6O_{17}$: J ber. 46,88%; gef. 46,55%.

Die Verbindung ist spielend leicht löslich in Wasser, wenig löslich in kaltem Methanol, sehr wenig löslich in Äthanol.

Beispiel 18

1,7-Bis-[N-{3,5-bis-(1,3-dihydroxyisopropylaminocarbonyl)-2,4,6-trijodphenyl}-N-(L-α-hydroxypropionyl)-amino]-4-oxaheptan

Formel II: $(HO)_2C_3H_5— = (HOCH_2)_2CH—$, $R = —CH_3$, Alkylen $= —CH_2CH_2CH_2—O—CH_2CH_2CH_2—$

56 g Na-Salz von IOPAMIDOL (0,07 Mol) in 100 g DMSO werden bei Raumtemperatur mit 12,4 g 1,7-Dijod-4-oxaheptan (0,035 Mol) umgesetzt, bis sich nach >90 Stunden die theoretisch erforderliche Menge Natriumjodid gebildet hat. Der Reaktionsverlauf kann durch titrieren von aliquoten Proben mit Silbernitrat verfolgt werden.

Man erhält 36,7 g der Titelverbindung, das sind 63,5% der Theorie.
Schmelzbereich: 228° sintern, 255° C Zersetzung.
DC: Rf = 0,16 und 0,24. Laufmittel Methylenchlorid/Methanol = 2 : 1.
$C_{40}H_{54}J_6N_6O_{17}$: J ber. 46,08%; 45,74%.
$[\alpha]_D^{20} = +17,9°$, $[\alpha]_{436}^{20} = +41,4°$ (c = 1,021% in Wasser).

Diese Verbindung ist spielend leicht löslich in Wasser, leicht löslich in Methanol, dagegen nur wenig löslich in Äthanol.

Beispiel 19

1,16-Bis-[N-{3,5-bis-(2,3-dihydroxypropylaminocarbonyl)-2,4,6-trijodphenyl}-N-hydroxyacetyl-amino]-4,7,10,13-tetraoxahexadecan

Formel II: $(HO)_2C_3H_5— = HOCH_2—CHOH—CH_2—$, $R = —H$,
Alkylen $= —CH_2CH_2CH_2—(OCH_2CH_2)_3—O—CH_2CH_2CH_2—$

39,25 g Na-Salz von 5-Hydroxyacetylamino-2,4,6-trijodisophthalsäure-bis-(2,3-dihydroxypropylamid) (0,05 Mol) in 60 g DMSO werden bei Raumtemperatur mit 12,16 g 1,16-Dijod-4,7,10,13-tetraoxahexadecan (0,025 Mol) unter Rühren umgesetzt bis sich die theoretisch geforderte Menge von Natriumjodid gebildet hat.

Man erhält 35 g der Titelverbindung, das sind 79% der Theorie.
Schmelzpunkt: 176—180° C.
DC: Rf = 0,18. Laufmittel Chloroform/Methanol = 1 : 1.
$C_{44}H_{62}J_6N_6O_{20}$: J ber. 43,35%; gef. 43,65%.

Diese Verbindung ist spielend leicht löslich in Wasser [≥100% (g/v) bei 20° C], leicht löslich in Methanol [12% (g/v)], wenig löslich in Äthanol und sehr wenig löslich in Chloroform.

Beispie 20

1,16-Bis-[N-{3,5-bis-(1,3-dihydroxyisopropylaminocarbonyl)-2,4,6-trijod-phenyl}-N-(L-α-hydroxypropionyl)-amino]-4,7,10,13-tetraoxahexadecan

Formel II: $(HO)_2C_3H_5— = (HOCH_2)_2CH—$, $R = —CH_3$,
Alkylen $= —CH_2CH_2CH_2—(OCH_2CH_2)_3—O—CH_2CH_2CH_2—$

40 g Na-Salz von IOPAMIDOL (0,05 Mol) in 60 g DMSO wird bei 20—25° C unter rühren mit 12,16 g 1,16-Dijod-4,7,10,13-tetraoxahexadecan (0,025 Mol) umgesetzt bis sich die von der Theorie geforderte Menge (0,05 Mol) Natriumjodid gebildet hat.

Man erhält 33,7 g der Titelverbindung, das sind 75% der Theorie.
Schmelzpunkt: 196—199° C.
DC: 0,28 und 0,35. Laufmittel Chloroform/Methanol = 2 : 1.
$C_{44}H_{66}J_6N_6O_{20}$: J ber. 42,67%; gef. 42,12%.
$[\alpha]_D^{20} = +15,6°$, $[\alpha]_{436}^{20} = +37,5°$ (c = 1,023% in Wasser).

Diese Verbindung ist spielend leicht löslich in Wasser [≥100% (g/v) bei 20° C], leicht löslich in Methanol, löslich in Äthanol, dagegen nur wenig löslich in Chloroform.

Beispiel 21

1,3-Bis-[N-{3,5-bis-(R(+)2,3-dihydroxypropylaminocarbonyl)-2,4,6-trijod-phenyl}-N-hydroxyacetyl-amino]-propan

Formel II: $(HO)_2C_3H_5- = R(+)HOCH_2-CHOH-CH_2-$, $R = -H$,
Alkylen $= -CH_2CH_2CH_2$

Diese Verbindung wird in genau gleicher Weise hergestellt, wie die im Beispiel 15 beschriebene racemische Verbindung.
Schmelzbereich: 224° sintern, 240° C Zersetzung.

$[\alpha]_D^{20} = +3,8°$, $[\alpha]_{436}^{22} = +9,2°$ (c = 5% in Wasser).

Die anderen Eigenschaften sind identisch mit denjenigen der racemischen Verbindung.

Das erforderliche Ausgangsmaterial 5-Hydroxyacetylamino-2,4,6-trijod-isophthalsäure-bis-(R(+)2,3-dihydroxypropylamid) wird nach einer ähnlichen Methode erhalten wie die im Beispiel 13 beschriebene racemische Verbindung.

63,5 g 5-Acetoxyacetylamino-2,4,6-trijod-isophthalsäuredichlorid gelöst in 90 ml DMAC werden in eine Lösung von 22 g R(+)2,3-Dihydroxypropylamin (0,242 Mol) in 45 ml DMAC in der noch 30,6 g Kaliumcarbonat (0,219 Mol) suspendiert sind, getropft. Man rührt 20 Stunden bei Raumtemperatur. Die weitere Behandlung und Aufarbeitung zum gewünschten 5-Hydroxyacetylamino-2,4,6-trijod-isophthalsäure-bis-(R(+)2,3-dihydroxypropylamid) erfolgt nach Beispiel 13.

Ausbeute 51,84 g, das sind 75% der Theorie.
DC: Rf = 0,22; Laufmittel: Äthylacetat/Eisessig/Wasser = 20 : 10 : 6.
$C_{16}H_{30}J_3N_3O_8$: J ber. 49,89%; gef. 50,04%.

## Verwendung

Unter den in den vorstehenden Beispielen beschriebenen Verbindungen werden im allgemeinen die Bis-[N-{3,5-bis-(2,3-dihydroxypropylaminocarbonyl)-2,4,6-trijod-phenyl}-N-hydroxyacetyl-amino]-alkane vorgezogen.

Sie sind vergleichsweise einfacher und billiger zugänglich als die 1,3-Dihydroxyisopropyl-Derivate, vor allem wenn diese zusätzlich α-Hydroxypropionyl-Reste am aromatischen Stickstoffatom aufweisen.

Die Wasserlöslichkeit der bevorzugten Verbindungen, die 2,3-Dihydroxypropyl- und Hydroxyacetyl-Reste aufweisen, ist praktisch unbeschränkt. Sie sind stabil. Sämtliche physikalisch chemischen und pharmakologischen Voraussetzungen für die Verwendung in Röntgenkontrastmitteln für die Neuroradiologie, im besonderen die Darstellung der Hohlräume, welche die Cerebrospinal-Flüssigkeit enthalten, werden durch diese Verbindungen besonders gut erfüllt.

Hervorzuheben ist insbesondere die niedrige Osmolalität der erfindungsgemäß erhaltenen Verbindungen, welche bedeutend niedriger ist als die der bisher praktisch verwendeten nicht ionogenen Röntgenkontrastmittel METRIZAMID und IOPAMIDOL, sowie die erhöhte Viskosität der neuen Kontrastmittel, welche besonders bei der Neuroradiologie das vorzeitige Abfließen des Kontrastmittels aus dem Inspektionsraum verhindert und bessere Kontrastaufnahmen ermöglicht.

In den folgenden Tabellen sind Osmolalität und osmotischer Druck und Viskosität von drei erfindungsgemäß erhaltenen Verbindungen A, B und C mit den zwei wichtigsten vergleichbaren vorbekannten, nicht ionogenen Röntgenkontrastmittel D und E verglichen.

Es bedeuten:

A = 1,3-Bis-[N-{3,5-bis-(2,3-dihydroxypropylaminocarbonyl)-2,4,6-trijodphenyl}-N-hydroxyacetyl-amino]-propan (Beispiel 15)

B = 1,5-Bis-[N-{3,5-bis-(2,3-dihydroxypropylaminocarbonyl)-2,4,6-trijodphenyl}-N-hydroxyacetyl-amino]-3-oxapentan (Beispiel 13)

C = 1,16-Bis-[N-{3,5-bis-(2,3-dihydroxypropylaminocarbonyl)-2,4,6-trijodphenyl}-N-hydroxyacetyl-amino]-4,7,10,13-tetraoxahexadecan (Beispiel 19)

D = L-5-α-Hydroxypropionylamino-2,4,6-trijod-isophthalsäure-bis-(1,3-dihydroxyisopropylamid) (Freiname [international non-proprietory name I. N. N.] = IOPAMIDOL)

E = 3-Acetylamino-5-N-methyl-acetylamino-2,4,6-trijodbenzoyl-glucosamin (Freiname [I. N. N.] = METRIZAMIDE)

Tabelle 1

| Verbindung | mg J/ml | Osmolalität (mOsm/kg) 37°C | Osmotischer Druck atm 37°C |
|---|---|---|---|
| A | 250 | 160 | 3,93 |
| | 300 | 184 | 4,67 |
| | 350 | 219 | 5,57 |
| B | 250 | 199 | 5,06 |
| | 300 | 240 | 6,11 |
| | 350 | 283 | 7,21 |
| C | 250 | 204 | 5,21 |
| | 300 | 269 | 6,84 |
| | 350 | 364 | 9,26 |
| D | 250 | 514 | 13,09 |
| | 300 | 619 | 15,76 |
| | 350 | 737 | 18,77 |
| E | 300 | 485 | |

Röntgenologen zur Verbesserung der Schattendichte und damit der Aussagekraft der Röntgenbilder so sehr gewünscht werden.

Der osmotische Druck der Verbindungen A und B ist sogar bei höchster Konzentration niedriger als derjenige der Körperflüssigkeiten (Blut=7,7). Mischungen von A, B und/oder C können bezüglich ihres osmotischen Druckes demjenigen der Körperflüssigkeiten genau angepaßt und dadurch mit geringster Belastung des Organismus verabreicht werden.

Das erlaubt die gefahrlose Anwendung von höchsten Dosen von Röntgenkontrastmitteln, die vom

Tabelle 2

| Verbindung | Viskosität in Centipoise (cP) wäßriger Lösungen enhaltend | | |
|---|---|---|---|
| | °C | 300 mg J/ml | 350 mg J/ml |
| A | 20 | 20,7 | 47,8 |
| | 37 | 7,4 | 17,3 |
| B | 20 | 19,5 | 38,5 |
| | 37 | 7,6 | 18,9 |
| C | 20 | 31,1 | 113,7 |
| | 37 | 16,5 | 34,9 |
| D | 20 | 8,95 | |
| | 37 | 4,70 | |
| E | 20 | 11,7 | |
| | 37 | 5,98 | |

Die vergleichsweise hohe Viskosität der neuen Röntgenkontrastmittel verhindert deren unerwünschte zu rasche Diffusion und damit Verdünnung des Kontrastes.

Die neuen Bis-[N-{3,5-bis-(hydroxyalkylaminocarbonyl)-2,4,6-trijod-phenyl}-N-(α-hydroxyacyl)-amino]-alkane der allgemeinen Formel (I) werden vorwiegend in Form ihrer wäßrigen Lösungen verwendet.

Es kommen je nach Verwendungszweck ca. 15 — ca. 70%ige Lösungen — g/v, d. h. 100%ig = 100 g Kontrastmittel/100 ml Lösung — mit einem Gehalt von etwa 60 bis ca. 450 mg J/ml zur Anwendung. Konzentrierte Lösungen werden bevorzugt. Die Art der Anwendung richtet sich nach dem sichtbar zu machenden Gefäß.

Für die Myelographie und Radiculographie werden die Lösungen nach lumbaler oder subokzipitaler Punktion instilliert. Bei der Ventriculorgraphie werden direkt die Ventrikel punktiert.

Dosierung:

| | |
|---|---|
| Myelographie | ca. 5—15 ml |
| Radiculographie | ca. 3— 5 ml |
| Ventriculographie | ca. 1— 2 ml |

Die Herstellung der Röntgenkontrastmittellösungen ist einfach, weil keine Salzlösungen bereitet werden müssen.

Beispielsweise werden die nach den vorstehenden Beispielen erhaltenen reinen 2,4,6-Trijod-isophthalsäureamide unter sterilen Bedingungen in der gewünschten Menge bidestilliertem Wasser gelöst, filtriert, in Serumflaschen oder Ampullen abgefüllt und anschließend sterilisiert. Die vorliegenden Trijod-isophthalsäureamide werden beim Hitzesterilisieren nicht zersetzt.

Beispiel 22

Injektionslösungen enthaltend 1,3-Bis-[N-{3,5-bis-(2,3-dihydroxypropylaminocarbonyl)-2,4,6-trijod-phenyl}-N-hydroxyacetyl-amino]-propan = Verbindung A

| Zusammensetzung von jeweils 20 ml Lösung | Gehalt der Injektionslösungen in mgJ/ml | | |
|---|---|---|---|
| | 200 | 300 | 350 |
| Verbindung A, g | 8,23 | 12,34 | 14,40 |
| Di-Na-Ca-Salz von Äthylendiamin-tetra-essigsäure-hexahydrat, mg | 5,2 | 7,8 | 9,0 |
| Tromethamin [Tris-(hydroxymethyl)-aminomethan], mg | 11,4 | 17,1 | 20 |
| Bidestilliertes Wasser bis, ml | 20 | 20 | 20 |
| Dichte bei 37°C, d | 1,208 | 1,319 | 1,371 |
| Viskosität bei 37°C, cP (cP = Centipoise) | 2,5 | 7,4 | 47,8 |

Ausführung: Das Na—Ca-Salz von Äthylendiamintetraessigsäure, das Tromethamin und das Kontrastmittel werden in bidestilliertem Wasser gelöst. Das pH der Lösung wird nötigenfalls durch Zusatz von 1 N Salzsäure auf ca. 7 eingestellt. Das Volumen wird auf 20 ml aufgefüllt. Die Lösung wird filtriert unter Verwendung einer Membrane von 0,45 mμ. Das Filtrat wird in Ampullen abgefüllt und während 30 Minuten bei 120° C sterilisiert.

Beispiel 23

Injektionslösung enthaltend Verbindung A und C:

| | |
|---|---|
| Verbindung A (s. Beispiel 15) | 50 g |
| Verbindung C (s. Beispiel 19) | 26,7 g |
| Natriumcarbonat | 0,4 g |
| Dinatriumsalz von Äthylendiamintetraessigsäure | 0,02 g |
| Bidestilliertes Wasser bis zum Volumen von | 100 ml |

Ausführung: Die Komponenten werden vereinigt, mit bidestilliertem Wasser auf 125 ml aufgefüllt, filtriert, unter hygenisch einwandfreien Bedingungen unter Stickstoff in Ampullen abgefüllt und anschließend sterilisiert; Jodgehalt: 350 mg/ml.

Beispiel 24

Infusionslösung

| | |
|---|---|
| Verbindung A (s. Beispiel 15) | 205,72 g |
| Natriumcarbonat | 0,5 g |
| Dinatriumsalz von Äthylendiamintetraessigsäure | 0,03 g |
| Bidestilliertes Wasser bis zum Volumen von | 500 ml |

Ausführung: Die Komponenten werden vereinigt, auf 500 ml aufgefüllt, filtriert, unter Stickstoff in 2 Infusionsflaschen abgefüllt und sterilisiert.

Jodgehalt: 200 mg/ml; Viskosität bei 20° C = 4,1 cP.

Beispiel 25

Injektionslösung

| | |
|---|---|
| 1,5-Bis-[N-{3,5-bis-(1,3-dihydroxyisopropylaminocarbonyl)-2,4,6-trijod-phenyl}-N-hydroxyacetyl-amino]-3-oxa-pentan (s. Beispiel 13) | 60,13 g |
| Di—Na—Ca-Salz von Äthylendiamintetraessigsäure · 6 $H_2O$ | 40 mg |
| Tromethamin | 47 mg |
| Bidestilliertes Wasser bis | 100 ml |

Ausführung: analog wie Beispiel 22; Jodgehalt: 300 mg/ml.

**Patentansprüche**

1. Bis-[N-{3,5-bis-(hydroxyalkylaminocarbonyl)-2,4,6-trijod-phenyl}-N-(α-hydroxyacyl)-amino]-alkane der allgemeinen Formel (I)

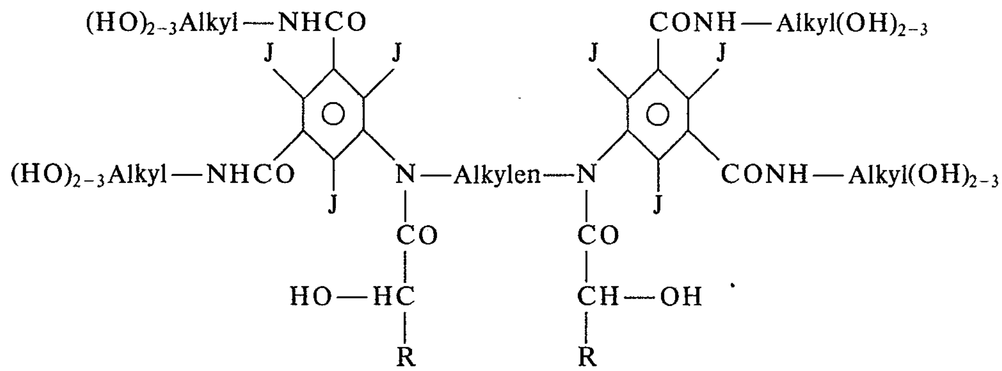

(I)

worin

| | |
|---|---|
| $(HO)_{2-3}$Alkyl- | 1,3-Dihydroxyisopropyl-, 2,3-Dihydroxypropyl- oder 1,3-Dihydroxy-2-hydroxymethylisopropyl-, |
| R | Wasserstoff oder Methyl- und |
| Alkylen | einen zweiwertigen Alkylen-Rest mit 2 bis 10 C-Atomen [$= -(CH_2)_{2-10}-$], der durch Hydroxy substituiert sein kann, oder einen Mono-, Di- oder Poly-oxa-alkylen-Rest mit 4 bis 12 C-Atomen [$= -(C_nH_{2n}-O)_{1-4}-C_nH_{2n}-$; $n=2$ oder 3], der durch Hydroxy substituiert sein kann, wobei die Hydroxygruppen jeweils mit einer niedrigen Fettsäure, vorzugsweise Essigsäure, acyliert oder im Falle von zwei oder mehr Hydroxygruppen durch Acetal- oder Ketalbildung, vorzugsweise mit Aceton, maskiert sein können, |

bedeutet.

2. Bis-[N-{3,5-bis-(dihydroxypropylaminocarbonyl)-2,4,6-trijod-phenyl}-N-(α-hydroxyacyl)-amino]-alkane nach Patentanspruch 1 der allgemeinen Formel (II)

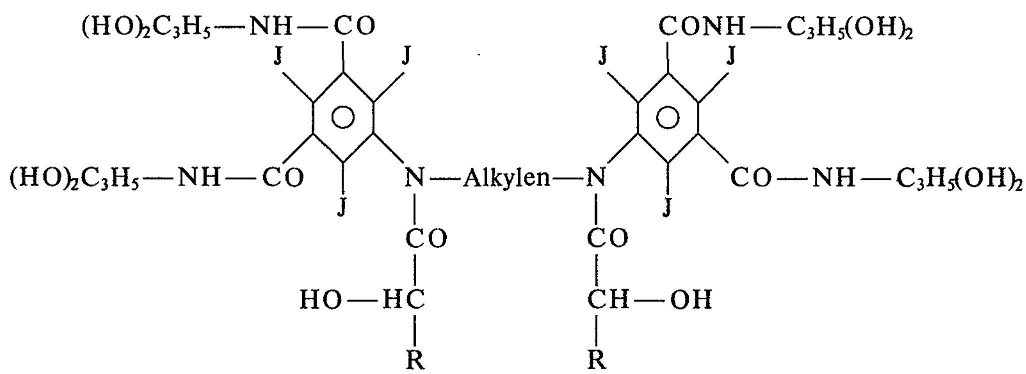

(II)

worin

| | |
|---|---|
| $(HO)_2C_3H_5$- | 1,3-Dihydroxyisopropyl- oder 2,3-Dihydroxypropyl-, |
| R | Wasserstoff oder Methyl- und |
| Alkylen | einen zweiwertigen Alkylen-Rest mit 2 bis 10 C-Atomen $[=-(CH_2)_{2-10}-]$, der durch Hydroxy substituiert sein kann, oder einen Mono-, Di- oder Poly-oxa-alkylen-Rest mit 4 bis 12 C-Atomen $[=-(C_nH_{2n}-O)_{1-4}-C_nH_{2n}-$; $n=2$ oder 3], der durch Hydroxy substituiert sein kann, wobei die Hydroxygruppen jeweils mit einer niedrigen Fettsäure, vorzugsweise Essigsäure, acyliert oder im Falle von zwei oder mehr Hydroxygruppen durch Acetal- oder Ketalbildung, vorzugsweise mit Aceton, maskiert sein können, |

bedeutet.

3. Verfahren zur Herstellung der als schattengebende Komponenten in Röntgenkontrastmitteln verwendbaren Bis-[N-{3,5-bis-(hydroxyalkylaminocarbonyl)-2,4,6-trijodphenyl}-N-(α-hydroxyacyl)-amino]-alkane nach Patentanspruch 1, dadurch gekennzeichnet, daß man ein 5-α-Hydroxyacylamino-2,4,6-trijod-isophthalsäure-bis(dihydroxypropylamid) der allgemeinen Formel (III)

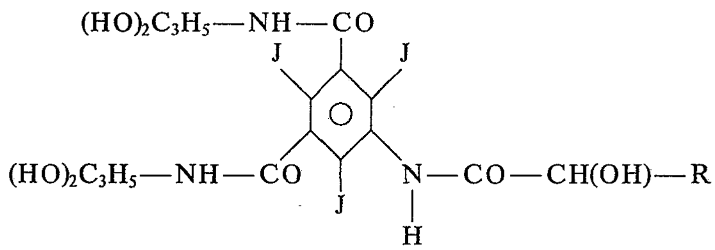

(III)

am an den Benzolring gebundenen Stickstoff-Atom im alkalischen Milieu alkyliert durch Umsetzung mit Alkylierungsmitteln der allgemeinen Formel (IV)

$$X-\text{Alkylen}-X \qquad (IV)$$

wobei R und Alkylen sowie $(HO)_2C_3H_5-$ in den Formeln (III) bzw. IV) die im Anspruch 1 definierten Bedeutung haben und X jeweils eines der Halogenatome Jod, Brom oder Chlor oder einen der Sulfat- bzw. Sulfonat-Reste $-OSO_2-OR_1$ bzw. $-OSO_2$-Alkyl oder $-OSO_2-$Aryl bedeutet.

4. Röntgenkontrastmittel, enthaltend als schattengebende Komponente mindestens ein Bis-[N-{3,5-bis-(hydroxyalkylaminocarbonyl)-2,4,6-trijod-phenyl}-N-(α-hydroxyacyl)-amino]-alkan gemäß Patentanspruch 1.

**Claims**

1. Bis-[N-{3,5-bis-(hydroxyalkylaminocarbonyl)-2,4,6-triiodo-phenyl}-N-(α-hydroxyacyl)-amino]-alkanes of the general formula (I)

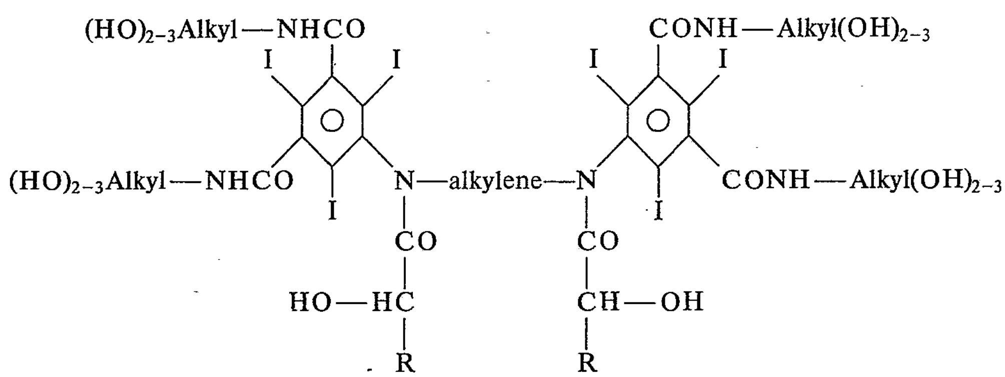

(I)

wherein $(HO)_{2-3}$alkyl-denotes 1,3-dihydroxyisopropyl, 2,3-dihydroxypropyl or 1,3-dihydroxy-2-hydroxymethylisopropyl, R denotes hydrogen or methyl, and alkylene denotes a divalent alkylene radical which has 2 to 10 C atoms $[=-(CH_2)_{2-10}-]$ and can be substituted by hydroxyl, or a mono-, di- or poly-oxa-alkylene radical which has 4 to 12 C atoms $[=-(C_nH_{2n}-O)_{1-4}-C_nH_{2n}-$; $n=2$ or 3] and can be substituted by hydroxyl, and each of the hydroxyl groups in the alkylene radicals can be acylated with a lower fatty acid, preferably acetic acid, or, in the

case of two or more hydroxyl groups, can be masked by acetal formation or ketal formation, preferably with acetone.

2. Bis-[N-{3,5-bis-(dihydroxypropylaminocarbonyl)-2,4,6-triiodo-phenyl}-N-(α-hydroxyacyl)-amino]-alkanes, according to Patent Claim 1, of the general formula (II)

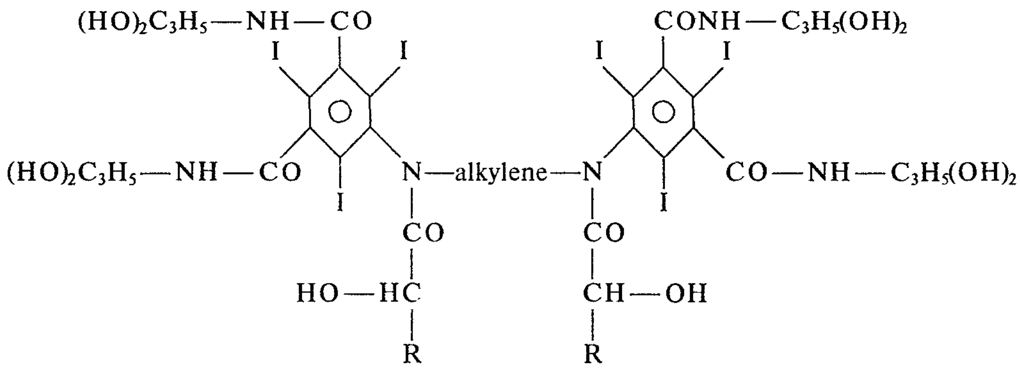

(II)

wherein $(HO)_2C_3H_5$ denotes 1,3-dihydroxyisopropyl or 2,3-dihydroxypropyl, R denotes hydrogen or methyl and alkylene denotes a divalent alkylene radical which has 2 to 10 C atoms [$= -(CH_2)_{2-10}-$] and can bei substituted by hydroxyl, or a mono-, di- or poly-oxa-alkylene radical which has 4 to 12 C atoms [$= -(C_nH_{2n}-O)_{1-4}-C_nH_{2n}-$; n=2 or 3] and can be substituted by hydroxyl, and each of the hydroxyl groups can be acylated with a lower fatty acid, preferably acetic acid, or, in the case of two or more hydroxyl groups, can be masked by acetal formation or ketal formation, preferably with acetone.

3. Process for the preparation of the bis-[N-{3,5-bis-(hydroxyalkylaminocarbonyl)-2,4,6-triiodo-phenyl}-N-(α-hydroxyacyl)-amino]-alkanes, according to Patent claim 1, which can be used as opacifying components in radiographic contrast media, characterised in that a 5-α-hydroxyacylamino-2,4,6-triiodo-isophthalic acid bis-(dihydroxypropylamide) of the genral formula (III)

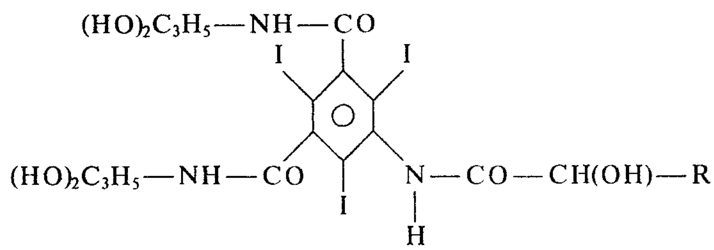

(III)

is alkylated, in an alkaline medium, at the nitrogen atom bonded to the benzene ring, by reaction with alkylating agents of the general formula (IV)

$$X-\text{alkylene}-X \quad \text{(IV)}$$

wherein R and alkylene, as well as $(HO)_2C_3H_5-$ in the formulae (III) or (IV), have the meaning defined in claim 1, and X in each case denotes one of the halogen atoms iodine, bromine or chlorine, or one of the sulphate or sulphonate radicals $-OSO_2-OR_1$ or $-OSO_2$-alkyl, or $-OSO_2-$aryl.

4. Radiographic contrast media containing, as the opacifying component, at least one bis-[N-{3,5-bis-(hydroxyalkylaminocarbonyl)-2,4,6-triiodo-phenyl}-N-(α-hydroxyacyl)-amino]-alkane according to Patent claim 1.

**Revendications**

1. Bis-[N-{3,5-bis-(hydroxyalkylaminocarbonyl-2,4,6-triiodo-phényl}-N-(alpha-hydroxyacyl)-amino]-alcanes de formule générale I

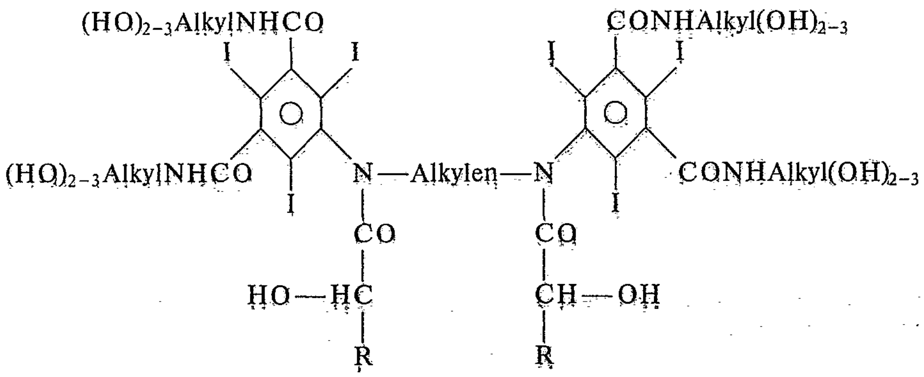

(I)

dans laquelle

(HO)2-3Alkyl- représente un groupe 1,3-dihydroxyisopropyle, 2,3-dihydroxypropyle ou 1,3-dihydroxy-2-hydroxyméthylisopropyle,

R représente l'hydrogéne ou un groupe méthyle et Alkylen représente un groupe alkylène divalent en C2—C10 [$= -(CH_2)_{2-10}-$], qui peut porter des substituants hydroxy, ou un radical mono-, di- ou poly-oxa-alkylène en C4—C12 [$= -(C_nH_{2n}-O)_{1-4}-C_nH_{2n}-$; n=2 ou 3], qui peut porter des substituants hydroxy, les groupes hydroxy en question pouvant être acylés par un acide gras inférieur, de préférence l'acide acétique ou bien, dans le cas de deux ou plusieurs groupes hydroxy, bloqués par acétalisation, de préférence à l'acide d'acétone.

2. Bis-[N-{3,5-bis-(dihydroxypropylaminocarbonyl)-2,4,6-triiodo-phényl}-N-(alpha-hydroxyayl)-amino]-alcanes selon la revendication 1, de formule générale II

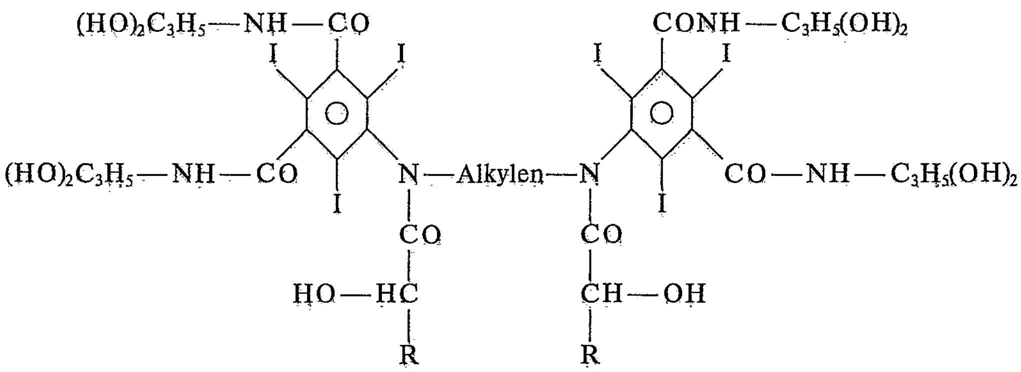

(II)

dans laquelle

$(HO)_2C_3H_5$- représente un groupe 1,3-dihydroxyisopropyle ou 2,3-dihydroxypropyle,

R représente l'hydrogène ou un groupe méthyle et Alkylen représente un groupe alkylène divalent en C2—C10 [$= -(CH_2)_{2-10}-$] qui peut porter des substituants hydroxy, ou un radical mono-, di- ou poly-oxa-alkylène en C4—C12 [$= -(C_nH_{2n}-O)_{1-4}-C_nH_{2n}-$; n=2 ou 3] qui peut porter des substituants hydroxy, les groupes hydroxy pouvant être acylés chacun par un acides gras inférieur, de préférence l'acide acétique, ou dans le cas de deux ou plusieurs groupes hydroxy, bloqués par acétalisation, de préférence à l'aide d'acétone.

3. Procédé de préparation des bis-[N-{3,5-bis-(hydroxyalkylaminocarbonyl)-2,4,6-triiodo-phényl}-N-(alpha-hydroxyacyl)-amino]-alcanes selon la revendication 1, utilisables en tant que composants opacifiants dans des agents de contraste radiologiques, caractérisé en ce que l'on alkyle un bis-(dihydroxypropylamide) d'acide 5-apha-hydroxyacylamino-2,4,6-triiodo-isophtalique de formule générale III

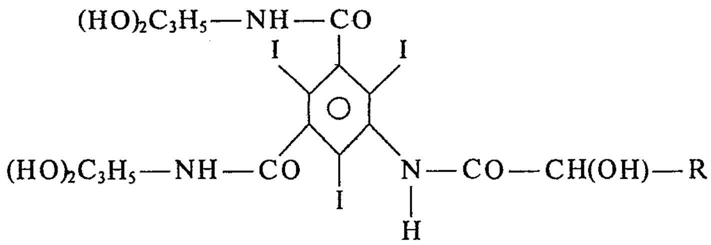

(III)

sur l'atome d'azote fixé sur le noyau benzénique, en milieu alcalin, par réaction avec des agents alkylants de formule générale IV

X—Alkylen—X (IV)

dans laquelle R et Alkylen et $(HO)_2C_3H_5$—, dans les formules III et IV respectivement, ont les significations indiquées dans la revendication 1 et X représente dans chaque cas l'un des atomes d'halogènes iode, brome ou chlore ou un des radicaux sulfates ou sulfonates $-OSO_2-OR_1$ ou $-OSO_2-$alkyle ou $-OSO_2-$aryle.

4. Agents de contraste radiologiques contenant en tant que composant opacifiant au moins un bis-[N-{3,5-bis-(hydroxyalkylaminocarbonyl)-2,4,6-triiodo-phényl}-N-(alpha-hydroxyacyl)-amino]-alcane selon la revendication 1.